(19) Europäisches Patentamt
European Patent Office
Office européen des brevets

(11) EP 2 150 629 B1

(12) **EUROPEAN PATENT SPECIFICATION**

(45) Date of publication and mention of the grant of the patent:
**13.08.2014 Bulletin 2014/33**

(21) Application number: **08758857.0**

(22) Date of filing: **29.05.2008**

(51) Int Cl.:
***C12Q 1/70*** *(2006.01)*

(86) International application number:
**PCT/EP2008/004277**

(87) International publication number:
**WO 2008/145366 (04.12.2008 Gazette 2008/49)**

(54) **IDENTIFICATION AND QUANTIFICATION OF ONCOGENIC HPV NUCLEIC ACIDS BY MEANS OF REAL-TIME PCR ASSAYS**

IDENTIFIZIERUNG UND QUANTIFIZIERUNG ONKOGENER HPV-NUKLEINSÄUREN MITTELS ECHTZEIT-PCR-TESTVERFAHREN

IDENTIFICATION ET QUANTIFICATION D'ACIDES NUCLÉIQUES VPH ONCOGÉNIQUES AU MOYEN D'ANALYSES PAR PCR EN TEMPS RÉEL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **01.06.2007 EP 07109454**

(43) Date of publication of application:
**10.02.2010 Bulletin 2010/06**

(73) Proprietor: **Hiantis S.r.l.**
**20146 Milano, (MI) (IT)**

(72) Inventors:
- **COCUZZA, Clementina, Elvezia, Anna**
  **I-20146 Milano (IT)**
- **BROCCOLO, Francesco**
  **I-20040 Busnago (MI) (IT)**

(74) Representative: **Predazzi, Valentina**
**Società Italiana Brevetti S.p.A.**
**Piazza di Pietra, 39**
**00186 Roma (IT)**

(56) References cited:
**WO-A-2004/031416**
**WO-A2-2006/063065**
**WO-A-2006/084155**
**US-A1- 2005 175 987**

- **PEITSARO PANU ET AL: "Integrated human papillomavirus type 16 is frequently found in cervical cancer precursors as demonstrated by a novel quantitative real-time PCR technique." JOURNAL OF CLINICAL MICROBIOLOGY MAR 2002, vol. 40, no. 3, March 2002 (2002-03), pages 886-891, XP002456424 ISSN: 0095-1137**
- **SUN ZHEN ET AL: "Multiplex locked nucleic acid probes for analysis of hepatitis B virus mutants using real-time PCR" GENOMICS, vol. 89, no. 1, January 2007 (2007-01), pages 151-159, XP002456516 ISSN: 0888-7543**
- **BROCCOLO FRANCESCO ET AL: "Additional evidence that pityriasis rosea is associated with reactivation of human herpesvirus-6 and-7" JOURNAL OF INVESTIGATIVE DERMATOLOGY, vol. 124, no. 6, June 2005 (2005-06), pages 1234-1240, XP002456425 ISSN: 0022-202X cited in the application**
- **TUCKER RUTH ANN ET AL: "Real-time PCR-based fluorescent assay for quantitation of human papillomavirus types 6, 11, 16 and 18" MOLECULAR DIAGNOSIS, NAPERVILLE, IL, US, vol. 6, no. 1, 1 March 2001 (2001-03-01), pages 39-47, XP002970543 ISSN: 1084-8592**
- **JOSEFSSON AGNETHA ET AL: "Detection and quantitation of human papillomavirus by using the fluorescent 5' exonuclease assay" JOURNAL OF CLINICAL MICROBIOLOGY, WASHINGTON, DC, US, vol. 37, no. 3, March 1999 (1999-03), pages 490-496, XP002193785 ISSN: 0095-1137**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SWAN DAVID C ET AL: "A sensitive, type-specific, fluorogenic probe assay for detection of human papillomavirus DNA" JOURNAL OF CLINICAL MICROBIOLOGY, WASHINGTON, DC, US, vol. 35, no. 4, April 1997 (1997-04), pages 886-891, XP002970544 ISSN: 0095-1137
- MOLDEN ET AL: "PreTect(TM) HPV-Proofer: Real-time detection and typing of E6/E7 mRNA from carcinogenic human papillomaviruses", JOURNAL OF VIROLOGICAL METHODS, ELSEVIER BV, NL, vol. 142, no. 1-2, 19 April 2007 (2007-04-19), pages 204-212, XP022034348, ISSN: 0166-0934, DOI: 10.1016/J.JVIROMET. 2007.01.036

## Description

The present invention refers to the identification and quantification of oncogenic HPV nucleic acids by means of Real-Time PCR assays.

Background of the invention

In recent years it has been established that infection by oncogenic human papillomavirus (HPV) is a necessary condition for cervical carcinogenesis (Zur Hausen, 2002). The most frequent high risk HPV types are HPV 16, -18, -31, -33, -45 and -58. Persistent infection is considered to be the true precursor of neoplastic progression (Kjaer et al., 2002). Currently, however, HPV infections are monitored primarily by qualitative HPV DNA detection assays which are often not type specific and therefore in the clinical management of the patients do not distinguish between persistent and transient infections, the latter being extremely frequent in sexually active women. Qualitative unspecific viral detection therefore represents an inefficient means of identifying women at risk of developing cervical cancer (Ho et al, 1998; Jacobs et al, 2000). There is therefore a need to establish a suitable clinical marker able to distinguish persistent from transient infection in order to better identify those women at risk of neoplastic progression. Thus, HPV detection and typing techniques have been proposed as an adjunct to, or a replacement for, the current cytological screening regime. Clearly the success of such strategies will depend on the development of rapid, reliable, sensitive, and specific HPV detection methods applicable in the clinical setting. HPV viral load has been proposed as a surrogate marker of persistent infection (Ho et al, 1998) but its use in identifying women at risk of developing cervical carcinoma (CC) remains controversial (Josefsson et al, 2000; Lorincz et al, 2002; Dalstein et al, 2003; Gravitt et al, 2003; Moberg et al, 2004).

Few reliable quantitative PCR assays have been developed for the measurement of HPV load and this could account for the discrepancy in the results obtained in previous studies. The lack of standardisation of the methods used, particularly the chosen HPV sequence to be amplified and the way the number of cells per sample is determined, may be responsible for the controversial results (Moberg et al. 2004). There is great interest in using validated quantitative HPV assays in epidemiological studies to better define the evolution of HPV infection and lesion progression. Several real-time PCR assays have been developed to measure HPV-16 DNA load by adjusting the signal obtained for HPV-16 DNA with the amount of cellular DNA calculated from amplification of a human gene (Swan et al., 1997, 1999; Josefsson et al., 1999, 2000; Ylitalo et al.,2000; Peitsaro et al., 2002; Nagao et al., 2002; Beskow and Gyllensten, 2002). In addition to viral load, also the integration state of the HPV genome is known to have profound implications for patient prognosis. However, whether viral load or integration status of HPV is a risk factor for cervical cancer progression remains unclear due to conflicting results obtained using different methodologies in previous studies.

Furthermore, several studies have shown that in cervical carcinogenesis, the expression of HPV of specific E6 and E7 oncogene transcripts is required for cell transformation and immortalisation. Moreover, the presence of E6 and E7 has been found to increase with increasing severity of cervical disease (Kraus et al., 2004, Molden et al 2005). Consequently, persistent expression of these oncogenes may serve as an indicator of progression to neoplastic precursor lesions and invasive cancer (Sotlar et al., 1998), Detection of E6 and E7 mRNA may therefore be an additional valid tool in adjunct to viral DNA load in order to identify patients who are more likely to undergo disease progression. There is currently one commercially available HPV assay, PreTect® HPV-Proofer (NorChip), which detects E6/E7 mRNA transcripts for HPV 16, 18, 31,33 and 45 using NASBA method (Molden et al 2007). Initial data, on the prognostic value and specificity of this method for underlying disease is promising (Kraus et al., 2004, Cuschieri et al., 2004, Molden et al 2005) but the clinical value of this method compared with the quantification of HPV-DNA assays remains to be determined.

A real-time PCR method for the quantitative and qualitative determination of oncogenic HPV to predict the risk of HPV infection resulting in cervical carcinoma is disclosed by Gyllesten et al, WO 2004/031416 A. The method allows viral load estimates for the range of HPV types most frequently found in different grades of cervical neoplasia and tumors. The individual species determination is performed using primers specific for the oncogenic E6/E7-transcripts of the HPV-types. A multiplex Real-Time PCR approach by which hepatitis B virus can be quantified by means of Sybr Green I dye is disclosed by Sun Zhen et al, Genomics, 89(1), 151-159, 2007.

Finally a better understanding of the circulating HPV genotypes in different geographical areas has become very important, particularly in view of the recent introduction of bivalent HPV vaccines (types 16 and 18). Screening programmes will in fact need to be continued particularly in areas where other oncogenic genotypes are found to prevalent, as immunization will only protect against HPV types targeted by the vaccine (Roden 2006 review).

## Description of the invention

The present invention provides a system enabling to detect one or more high-risk HPV genotypes in clinical samples and to determine viral oncogenic activity by means of both specific DNA load and presence of E6/E7 mRNA.

This system minimizes the number of parallel reactions performed for each sample and makes it suitable for use in routine screening of biological specimens such as cervical cytological samples, peripheral blood, urine, tissue biopsies and the like.

The invention more particularly provides a method for the identification and quantification of oncogenic HPV nucleic acids by means of Real-Time PCR assays comprising:

1) first line screening by means of 5 independent SYBR Green I Real-time PCR assays to determine the total viral load and to identify the presence of one or more of 13 high risk HPV genotypes in the sample;
2) second line assays to be applied to samples which have proved positive to first line screening, including:

- 5 independent TaqMan Real-time PCR assays to determine the presence and the viral load of the most common oncogenic HPV types: 16, 18, 31, 45, 33 group (including 33, 52, 58, 67 genotypes).
- 6 independent SYBR Green I RT Real-time PCR assays to determine the presence in the sample of the oncogenic transcripts E6/E7 of HPV types 16, 18, 31, 33, 45, 58.

<u>Detailed description of the invention</u>

The invention provides a method for the identification and quantification of oncogenic HPV nucleic acids comprising:

a) first line screening by means of 5 independent SYBR Green I Real-time PCR assays to determine the total viral DNA load and to identify the presence of one or more of 13 high risk HPV genotypes in clinical samples;
b) second line assays to be applied to samples which have proved positive to first line screening, including:

i) 5 independent TaqMan Real-time PCR assays to determine the presence and the viral load of the most common oncogenic HPV types: 16, 18, 31, 45, and 33 group (including 33, 52, 58, 67 genotypes).
ii) 6 independent SYBR Green I RT Real-time PCR assays to determine the presence in the sample of the oncogenic transcripts E6/E7 of HPV types 16, 18, 31, 33, 45, 58.

Real-Time PCR assays in virology are known and reviewed for instance by Mackay et al., 2002, Nucleic Acid Research, 30(6), 1292-1305.

Any clinical specimen obtainable from human tissues, organs or fluids may be used in the method of the invention.

Primers for the first SYBR Green I Real-Time PCR assay (step a) are reported below.

**TABLE 1**

| GENOTYPE | FORWARD | REVERSE |
|---|---|---|
| High-risk HPV | CGTCCAAAAGGAAACTGAGC (SEQ ID NO. 1) | GCACAGGGACATAACAATGG (SEQ ID NO: 2) |
| HPV-16 | CGAAAGTATTTGGGTAGTCCACTTA (SEQ ID NO. 3) | CAGCTCTACTTTGTTTTTCTATAC ATATGG (SEQ ID NO: 4) |
| HPV-18/45 | TTTGAAAGGACATGGTCCAGAT (SEQ ID NO 5) | CGTTCCGAAAGGGTTTCC (SEQ ID NO: 6) |
| HPV-31 | CCACCACATCGAATTCCAA (SEQ ID NO. 7) | CGCCGCACACCTTCAC (SEQ ID NO: 8) |

The preferred primers for Real-time PCR (SYBR Green I method) for the determination of oncogenic transcripts E6/E7 (step b-ii) are reported below:

**TABLE 2**

| GENOTYPE | FORWARD | REVERSE |
|---|---|---|
| HPV 16 | CAGAGCTGCAAACAACTATACAT GATATA (SEQ ID NO: 9) | GTTAATACACCTCACGTCGCAGTA (SEQ ID NO: 10) |
| HPV 18 | TTACAGAGGTGCCTGCGGT (SEQ ID NO: 11) | TCTAAGTTTTTCTGCTGGATTCAAC (SEQ ID NO 12) |
| HPV 31 | CATTGGAAATACCCTACGATGAA (SEQ ID NO: 13) | TTGACACGTTATACACCTTTGCAG (SEQ ID NO. 14) |

(continued)

| GENOTYPE | FORWARD | REVERSE |
|---|---|---|
| HPV 33 | TTGAACTACAGTGCGTGGAATG (SEQ ID NO: 15) | CAGCGCCCTCAGATCGTT (SEQ ID NO 16) |
| HPV 45 | ACAAGACGTATCTATTGCCTGTG TATAT (SEQ ID NO: 17) | CCGCAGGCACCTCTGTG (SEQ ID NO: 18) |
| HPV 58 | ATCGAATTGAAATGCGTTGAA (SEQ ID NO 19) | GCACAGCGCCCTCAGAT (SEQ ID NO: 20) |

Finally, the preferred primers for the TaqMan Real-Time PCR assays (step b-i) are reported in table 3 of the following Experimental Section #1.

**Experimental Section #1**

MATERIALS AND METHODS

*Sample preparation from cervical samples*

Cervical cytological material was scraped from the endocervix using a rotary motion with a Cytobrusch (Digene Cervical sampler, Digene Corp., Gaithersburg, MD, USA) and then introduced in collection devices and rinsed into a vial containing 10 ml of phosphate-buffered saline (pH 7.4). Specimens were refrigerated and transported to the microbiology and virology laboratory within 1 hr on wet ice where they centrifuged at 1800 rpm for 10 min, and stored as split cellular pellets at -70°C prior to nucleic acid extraction and HPV detection. Two different extraction methods (manual and automated) were compared in this study. Manual method includes organic extraction (phenol-chloroform) of the samples. Briefly, cellular pellets were resuspended in 450 μl of lysis solution containing 100 mM KCl, 10 mM Tris-HCl (pH 8.3), 2.5 mM $MgCl_2$, 0.5% (vol/vol) Tween 20, and 0.5% (vol/vol) Nonidet P-40. Samples were incubated at 95°C for 30 min, mixed for 2 min, and digested with 50 μl of proteinase K (20 μg/μl). After overnight incubation at 56°C, samples were heated at 95°C for 10 min to inactivate proteinase K. Phenol-chloroform extraction followed by high-salt isopropanol precipitation was performed as previously described (18), and purified material was resuspended in a final volume of 200 μl of AE buffer (5 mM Tris-HCl-0.5 mM EDTA). Automated method, a commercial kit for DNA extraction based on binding of nucleic acid to silica membrane (Macherey-Nagel Nucleospin robot 96 blood kit) was used. The 96-well plate format of the commercially available Macherey-Nagel Nucleospin kit allow integration into the workstation of a robotic liquid handler (Biomeck 2000, Beckman). To enable further automation of the procedure a set of 48 frozen cervical cytological samples previously tested for HPV-16 (24 samples HPV-16 positive and 24 HPV-16 negative), each divided into two pellets of equal aliquots, were extracted by the two methods.

Briefly, 200 μl of the resuspended cellular pellet, 25 μl of proteinase K, and 200 μl of Nucleospin lysis buffer BQ1 were added to each other and vortexed for 30 s.

To maximise DNA recovery from difficult samples (i.e. samples containing much emoglobin) the sample was then incubated for at least 1 hour at 56°C. After incubation, 200 μl of 96 to 100% ethanol was added to each sample and the mixtures were vortexed again.

The Nucleospin plate was placed on top of the vacuum manifold, and the samples were distributed to the appropriate wells. A vacuum of 400 mbar was applied for 5 min or until the samples had been completely drawn through the filter. Each well was washed by adding 300 μl of Nucleospin B5 washing buffer. A vacuum of 400 mbar was then applied for 5 min, and the through-flow was discarded. Then 600 μl of Nucleospin buffer B5 was added to each well, and the vacuum was applied at 400 mb for 3 min. The vacuum was applied at 600 mbar for a further 10 min to remove any residual ethanol. The DNA was eluted by adding 200 μl of Nucleospin elution buffer BE directly to the silica membrane in each well. The plate was incubated with this solution at room temperature for 5 min, and then a vacuum of 400 mbar was applied for 10 min to elute the DNA.

*Quantification of HPV DNA by Real-time quantitative PCR assays*

Plasmids containing HPV-16, -18, -31, -33, -45, -52, -58 and -67 sequences were prepared by cloning from PCR products of clinical samples and a standard curve for each assay was set up. PCR products were cloned into the pCRII plasmid by using the TOPO-TA cloning kit (Invitrogen Corp., San Diego, Calif.) according to the manufacturer's instructions. The plasmids with integrated HPVs were purified with the Qiagen plasmid Maxiprep kit (Qiagen, Inc.), sequenced, and used to quantify the HPV-16, -18, -31, -33, -45, -52, -58 and -67 DNA.

Five independent real-time quantitative TaqMan PCR assays: HPV-16, 18, 31, 45 and 33 group (33, 52, 58 and 67 genotypes are indistinguishable from each other). In order to normalize the HPV viral load to the number of cells in the sample, a quantitative detection system a single-copy human CCR5 gene was also used (Broccolo et al., 2005). Cervical samples differ widely in the amount of DNA present. DNA from cervical samples was considered suitable for HPV viral load determination if the human CCR5 copy number for reaction was higher than $2x10^3$ (corresponding to $10^3$ cells for reaction). Amplification and detection were performed using using TaqMan technology and ABI Prism device (7900 SDS; Applied Biosystems, Forster City, CA). All reactions were optimized to obtain the best amplification kinetics under the same cycling conditions (2 min at 50°C, 15 min at 95°C, and 40 cycles of 15 s at 95°C and 1 min per cycle at 60°C) and composition of the reaction mixture. All reactions were performed in a final volume of 25 μl containing 100 mM (each) dATP, dCTP, and dGTP; 200 mM dUTP; 4 mM MgCl2; 1 X TaqMan buffer A (Applied Biosystems Foster City, Calif); 0.625 U of AmpliTaq Gold, 0.25 U of uracil-N-glycosylase; and 10 μl of DNA template. Tubes, containing all PCR components but without template DNA (denoted NTC reaction), were used to ensure that the reagents mix were free of contamination. Target-specific primers and probes were used at the final concentrations of 300 and 200 nM, respectively. The results of the experiment using these conditions are shown for HPV-16, HPV-33 and -58 in figure 1 and 2, respectively. The principle of the real-time PCR has been described elsewhere (Heid, et al. 1996). Briefly, the fluorescent signal (Rn) generated by the degradation of the hybridized probe is automatically calculated by a computer algorithm that normalizes the reporter emission signal first; by dividing it by the emission of a control dye (ROX) present in the PCR mix and then by subtracting all the background signals generated in the first 15 cycles of PCR. Then, the algorithm calculates the cycle of threshold (Ct) at which each PCR amplification reaches a threshold value (usually set at 10 times the standard deviation of the baseline signal) that is inversely proportional to the log number of target copies present in the sample. A standard curve was drawn using serial dilutions of known input target copies (x axis) versus the corresponding Ct values (y axis) using the least-squares fit method.

*Primer and probe design*

The target sequences for HPV-16 and 33 group are in the E1 open reading frame (ORF); sequences for HPV-18 and 45 are in the E6 ORF, while sequences for HPV-31 is in the E2 ORF. The primers and TaqMan probes (Table 3) were designed using Primer Express software (PE Biosystem, Foster City, Calif.).

**TABLE 3.** Primers and fluorescently labelled hybridization probes used for the quantification of DNA of HPV-16, -18, -31, -33, -45, -52 -58 and -67 by Real-time PCR.

| Target (Accession Number) | Sequence (5' to 3') | Labels (5', 3') Fluorophore |
|---|---|---|
| HPV-16 (NC_001526) | F16 (SEQ ID NO 3) R16 (SEQ ID NO 4) Probea 16 (SEQ ID NO 21): 5'-AGTGAATGTGTAGACAATAA-3' | VIC, none |
| HPV-18 (AY863183) | F18 (SEQ ID NO 22): 5'-ttttgctgtgcaaccgatt-3' R18 (SEQ ID NO 23): 5'-agtgccagcgtactgtattgtg-3', Probe[a] 18 (SEQ ID NO 24): 5'-CGGTTGCCTTTGGCTT- 3' | FAM, none |
| HPV-31 (J04353) | F31 (SEQ ID NO 7) R31 (SEQ ID NO 8) Probe[b] 31 (SEQ ID NO 25): 5'-CCTGCGCCTTGGGCACC-3' | VIC, TAMRA |
| HPV-45 (EF202167) | F45 (SEQ ID NO 26): 5'-CAGTACCGAGGGCAGTGTAA-3' R45 (SEQ ID NO 27): 5'-cgtctgcgaagtctttcttg-3' Probea 45 (SEQ ID NO 28): 5'-acatgttgtgaccaggc-3' | VIC, none |
| HPV-33/52/58/-67 (M12732) | F 33group (SEQ ID NO 29) 5'-GGACGTGGTGCAAATTAGATTT-3' R 33 group (SEQ ID NO 30) 5'-GTGCTGATATTTCCTCCATGGT-3' Probea 33 group (SEQ ID NO 31) 5'-AGGAAGAGGACAAGGAA-3' | FAM, none |
| [a]MGB Probe: minor groove binder; [b]TAMRA probe | | |

As shown in table 3, two types of fluorogenic hybridization probes were utilized: one dual-labelled probe (in the assay to quantify HPV-31) and four single-labelled probes (in the assays to quantify HPV-16, 18, 45 and 33 group). The single-labelled probes have a minor groove binder (MGB) and non-fluorescent quencher at the 3'-end of the DNA sequence whereas dual-labelled probes have a fluorescent quencher (TAMRA) at the 3'-end of the DNA sequence. HVP-18 and 33 group probes were labelled to the 5'-end of the DNA sequence with 5' fluorophore (FAM), while HPV-16, 31 and 45 with 5' fluorophore (VIC). Finally, all sequences found in GenBank for each HPV genotype studied were aligned and primers and probe were chosen to avoid the mismatch between variants; the alignment was performed to select a highly conserved region for each virus. Each primer and probe selected was checked against the alignment to ensure that each primer and probe was targeted to a conserved region in the respective alignments.

*HPV typing by GP+-PCR system and INNO-LIPA analysis*

A total of 296 samples were tested for the presence of HPVs using two PCR primer set GP5+/GP6+ (GP+-PCR); the limit of sensitivity for all genotypes studied was $10^4$ copies for reaction (data not shown). The HPVs types detected using consensus primers were identified by sequencing of the PCR product using a fluorescently labeled dideoxy terminator kit (Amersham Pharmacia Biotech, Little Chalfont, United Kingdom), The sequences generated were compared to HPV sequences at GeneBank using the Fasta program (program manual for the Wisconsin package; Genetics Computer Group, Madison, Wis.). Next, a total of 15 samples that fall within the sensitivity range of $GP^+$-PCR system but that were detected only by real-time PCR assays were also tested by PCR using the consensus primers sets MY09/MY11 (MY-PCR); the conditions of the $GP^+$-PCR and MY-PCR systems were performed as described previously (Jacobs et al., 1995; de Roda Husman et al., 1995; Qu et al., 1997). Subsequently, the samples MY-PCR positive were identified by sequencing of the PCR product. Another set of 31 samples were tested for the presence of HPVs using the INNO-LiPA HPV Genotyping v2 test (Innogenetics, Ghent, Belgium), following manufacturer's instructions. The INNO-LiPA HPV Genotyping v2 test enables specific detection of 24 HPV types, namely types -6, -11, -16, -18, -31, -33, -35, -39, -40, -42, -43, -44, -45, -51, -52, -53, -54, -56, -58, -59, -66, -68, -70, and -74, including multiple combinations. The test is based on PCR amplification of a 65 bp fragment within the L1 region of the HPV genome using the broad spectrum SPF10 biotinylated primers. Biotinylated amplicons are subsequently hybridized with HPV type-specific oligonucleotide probes which are immobilized as parallel lines on membrane strips. After hybridization and stringent washing, streptavidin-conjugated alkaline phosphatase is added and bound to any biotinylated hybrid formed. Incubation with BCIP/NBT chromogen yields a purple precipitate and the results can be visually interpreted (Kleter et al., 1999).

*Statistical analysis*

Accuracy, defined as the level of approximation of a measured value to a reference value taken as a "gold standard," was estimated by computing the arithmetic differences between DNA copy number evaluated by the real-time PCR assay and the theoretical number inferred from UV spectroscopy. The significance of systematic biases was assessed by a paired sample Student t test and was adjusted by covariance analysis, when needed. Repeatability and reproducibility were estimated by computing the coefficient of variation (CV) (the ratio between the standard deviation and the mean of repeated measurements) under different conditions. The repeatability and reproducibility of the TaqMan assays were assessed by calculating the CVs of the copy numbers determined experimentally experimentally (180 measurements for each reference DNA). The difference between reference curves was assessed by covariance analysis. Normalization of HPV type-specific viral load was calculated as:

$$VL = \frac{Cn_{HPV}}{(Cn_{CCR5}/2)} \times 10^4 \text{ cells}$$

where VL is the number of HPV genomes per $10^4$ cells (corresponding to 2x $10^4$ CCR5 copies, $Cn_{HPV}$ is the number of HPV genomes and $(Cn_{CCR5}/2)$ is the number of cells (corresponding to CCR5 copies number x 2).

Statistical analyses were performed by using the SPSS statistical package (SPSS, Inc., Chicago, Ill.). The kappa statistic was calculated to evaluate the agreement between rates of HPV positivity. Kappa statistics less than 0.4 represents fair too poor agreement, values of 0.4 to 0.8 represents moderate to good agreement, and values of more than 0.8 represent excellent agreement.

**Results**

*Rationale and design of the typing system*

The present typing system was designed in order to allow quantitation of the viral load for those high-risk HPV genotypes most frequently associated with cervical intraepithelial carcinoma and neoplasia. Although the prevalence of the oncogenic HPV types varies between studies, in the design of this typing system we focused on HPV-16, -18, -31, -33, -45, -52, -58 and -67. Five independent quantitative PCR fluoroscent 5'-exonuclease reactions were designed and optimized using four separate aliquots of the same DNA specimen. The positions of the primers and probes chosen for these assays are shown in Table 3.

*Strategies in probe and primers design*

To maximize sensitivity, DNA targets for real-time PCR amplifications were <100bp and the sequence of the probe was chosen so as to be close to the 3'forward primer (no further than 5 nt). To reduce the number of assays, the primers and probes were chosen so as to quantify in the same reaction tube HPV-33, and -52, -58 and -67 (HPV-33/-52/-58/-67 TaqMan assay); HPV-33 genotype sequence was chosen as reference for the assay used to amplify HPV-33 and -52, -58, -67, (table 4). Thus, the HPV-33 group TaqMan assay allows the quantification of either HPV-33, -52, -58, -67, or combinations of these. Shorter MGB probes were chosen for the detection of HPV-33 and/or -52, -58, -67 as well as for HPV-16, 18, 31 and 45. This was found to be necessary in order to avoid the presence of mismatches between the sequences of HPV-33/-52/-58/-67 as well as polymorphisms within sequences of different HPV-16, 18, 31 and 33 group variants. By contrast, for HPV-31 a longer dual-labeled probe was also chosen as no mismatch were found in the probe among HPV-31 subtypes present in GenBank. The presence and localization of mismatches in the assay used to amplify HPV--33, -52, -58, -67 are reported in table 4. Furthermore, special attention was taken in choosing sequences when there were found to be present one or more mismatches; in particular it was avoided to have in both probes and primers polymorphisms close within the same sequence and polymorphisms at the 5' end.

**Table 4**. Localization of mismatches for HPV-52/-58/-67 using HPV-33 as reference sequence

| Genotypes | Presence of mismatches | | |
|---|---|---|---|
| | Primer forward | probe | Primer reverse |
| HPV-33 vs. -52 | - | - | 1 |
| HPV-33 vs. -58 | 2 | - | 1 |
| HPV-33 vs. -67 | 1 | - | 0 |

*Dynamic range and analytical sensitivity and specificity*

In order to generate reference curves for the determination of HPV-16, -18, -31, -33, -45, -52, -58 and -67 viral loads (quantitation), plasmids were quantified by UV spectroscopy. Thereafter, three distinct sets of 10-fold dilutions for each construct were prepared and amplified by PCR in the same run of the 7900 ABI Prism sequence detector system. A wide dynamic range characterized both assays, discriminating between $10^0$ and $10^6$ HPV genome equivalents/reaction. For all the systems generated, a strong linear relationship between the log of the starting copy number and the *Ct* values was obtained ($r^2$ = 0.99) (Fig. 1).

To better mimic the situation in which DNA is extracted from cells, we performed some experiments by adding human HPV-negative genomic DNA (100 ng of cervical cells DNA corresponding to 15,000 cellular genome equivalents) to a wide range of HPV-16 template DNA ($10^0$ to $10^6$ copy equivalent of DNA). As shown in table 1, the sensitivity and the performance of the assay were not affected by the presence of 100 ng of human genomic DNA. Standard curves ranging from $10^1$ to $10^6$ copies per sample were constructed for each of the HPV types, or groups of HPV types, based on 12 independent measurements for each HPV copy number. Similar results were obtained for all others amplifications (data not shown). By contrast, we observed that the sensitivity was affected when adding an excess of human HPV-negative genomic DNA (1 μg of cervical cells DNA corresponding to 150,000 cellular genome equivalents) to each reference standard ($10^1$ to $10^3$ copy equivalent of DNA). To resolve this problem we increased by 5 sec in a stepwise fashion each anneling/extension step for all cycles (table 5). However, when concentration of human genomic DNA was higher (>1 μg) the sample was diluited in order to have a final concentration not higher (equal or lower than) 1 μg/reaction (no higher that 1 μg for reaction).

**Table 5.** Sensitivity for each reference standard in presence and in absence of genomic DNA

| TaqMan PCR Assays | In absence of increase of the anneating/extension | | | In presence of increase of the annealing/extension | |
|---|---|---|---|---|---|
| Reference standard | Plasmid in absence of human DNA | Plasmid diluited in 100 ng human DNA | Plasmid diluited in 1 μg human DNA | Plasmid diluited in 100 ng human DNA | Plasmid diluited in 1 μg human DNA |
| Copy no./reaction | Mean Ct | | | Mean Ct | |
| TaqMan Assay for HPV-16 | | | | | |
| $10^1$ | 36.41 | 36.58 | 39.86 | 36.51 | 36.70 |
| $10^2$ | 33.10 | 33.18 | 34.05 | 33.15 | 33.38 |
| $10^3$ | 29.80 | 29.86 | 30.15 | 29.81 | 29.85 |
| TaqMan Assay for HPV-18 | | | | | |
| $10^1$ | 35.53 | 35.65 | 39.95 | 36.55 | 35.89 |
| $10^2$ | 32.20 | 32.26 | 34.05 | 33.21 | 32.48 |
| $10^3$ | 28.90 | 28.98 | 30.15 | 29.92 | 29.85 |
| TaqMan Assay for HPV-45 | | | | | |
| $10^1$ | 36.60 | 36.82 | 40.00 | 36.81 | 36.82 |
| $10^2$ | 33.28 | 33.31 | 30.84 | 33.30 | 33.15 |
| $10^3$ | 29.98 | 30.05 | 31.02 | 29.96 | 29.91 |
| TaqMan Assay for HPV-31: | | | | | |
| HPV-31 | | | | | |
| $10^1$ | 36.41 | 36.81 | 39.86 | 36.75 | 36.92 |
| $10^2$ | 32.73 | 33.52 | 34.05 | 33.50 | 33.58 |
| $10^3$ | 29.32 | 30.11 | 30.15 | 30.10 | 30.15 |
| TaqMan Assay for HPV-33 group: | | | | | |
| HPV-33 | | | | | |
| $10^1$ | 35.63 | 35.98 | 39.86 | 36.00 | 35.98 |
| $10^2$ | 32.29 | 32.36 | 34.05 | 32.35 | 32.45 |
| $10^3$ | 29.01 | 29.12 | 30.15 | 29.08 | 29.15 |
| HPV-52 | | | | | |
| $10^1$ | 35.72 | 36.01 | 39.91 | 35.81 | 36.02 |
| $10^2$ | 32.40 | 32.54 | 34.15 | 33.45 | 32.68 |
| $10^3$ | 29.02 | 29.12 | 30.25 | 29.11 | 29.23 |
| HPV-58 | | | | | |
| $10^1$ | 36.63 | 36.91 | 40.00 | 36.68 | 36.82 |
| $10^2$ | 33.38 | 33.62 | 34.85 | 32.89 | 33.08 |
| $10^3$ | 30.01 | 30.23 | 31.38 | 29.46 | 29.75 |
| HPV-67 | | | | | |
| $10^1$ | 35.63 | 36.81 | 39.96 | 35.63 | 36.81 |
| $10^2$ | 32.29 | 33.32 | 34.21 | 32.29 | 33.32 |
| $10^3$ | 29.01 | 30.11 | 30.36 | 29.01 | 30.11 |

The specificity was tested by determining the ability of primer and probe combinations to discriminate plasmids with different HPV types. No aspecific signal was observed for the indipendent real-time PCR assays (data not shown).

In addition, the kinetics profile of the two assays for the amplification of HPV-18 and -45 genotypes was compared; no significant differences were found between the plots generated for the two constructs (F = 1.30 [P = 0.23] and 1.35 [P = 0.22] for the intercept and the slope of the plots, respectively) (Fig. 2A); similar results were obtained for the amplification of HPV-33, -52, -58 and -67 genotypes (Fig. 2B).

*Accuracy, repeatability and reproducibility*

Next, we measured the accuracy error, the intra- and inter-experimental variability for each TaqMan PCR assay (table 6). In the table 6, we showed the data only obtained with the HPV-16, -18,-31 and -33 reference standard; however, similar results were obtained for reference standard containing others HPV genotypes detected by two assays (HPV-45 and HPV-52, -58 and -67).

**Table 6.** Accuracy error, repeatability and reproducibility of TaqMan assays for quantitation of HPV-16, -18,-31 and -33 group

| | TaqMan Assay for HPV-16 | | | TaqMan Assay for HPV-18 | | | TaqMan Assay for HPV-31 | | | TaqMan Assay for HPV-33/-52/-58/-67 | | |
|---|---|---|---|---|---|---|---|---|---|---|---|---|
| Copy no. | | CV (%) | | | CV (%) | | | CV (%) | | | CV (%) | |
| | Accuracy Error (%), mean ± **SE** | Intra-assay | Inter-assay | Accuracy Error (%), mean ± SE | Intra-assay | Inter-assay | Accuracy Error (%), mean ± SE | Intra-assay | Inter-assay | Accuracy Error (%), mean ± SE | Intra-assay | Inter-assay |
| $10^1$ | -9±19 | 30 | 46 | -14 ± 4.7 | 29 | 44 | -16 ±9 | 42 | 71 | -21 ± 9 | 42 | 71 |
| $10^2$ | 14±9 | 16 | 24 | -13 ± 2.9 | 14 | 21 | -14 ± 5 | 18 | 39 | -17 ± 5 | 18 | 39 |
| $10^3$ | -4 ± 2 | 8 | 21 | -16 ± 2.4 | 7 | 18 | -8 ± 2 | 15 | 36 | -9 ± 2 | 15 | 36 |
| $10^4$ | -3±3 | 7 | 20 | -12 ± 2.7 | 6 | 23 | 5 ± 3 | 8 | 34 | 6 ± 3 | 8 | 34 |
| $10^5$ | 3±3 | 7 | 16 | 2 ± 2.8 | 7 | 20 | 2 ± 3 | 6 | 28 | 3 ± 3 | 6 | 28 |
| $10^6$ | 2±1 | 5 | 9 | 5 ± 1.5 | 5 | 9 | 6 ± 2 | 5 | 17 | 7 ± 2 | 5 | 17 |

Automated nucleic acid extraction by Biomeck 2000

To enable further automation of the procedure a set of 48 frozen cervical cytological specimens (each divided into two pellets obtained following centrifugation of two equal volumes of sample), were extracted by two different methods: manual extraction (phenol-chloroform protocol) and automated extraction (using an extraction kit integrated into a workstation of a robotic liquid handler). All steps (reagent mixing, incubation, washing and elution) excluding the loading of the sample and pellet lysis were performed by an automated station (Biomeck 2000 instrument).

Real-time PCR assays were carried out to compare the quality of DNA obtained by the Machery Nagel extraction method with that recovered by the classical phenol-chlorophorm extraction. The kinetics of the amplification plots showed no evidence of the presence of inhibitors in the DNA extracted by both methods (data not shown). Twenty-four strongly positive for HPV-16 and 24 HPV negative samples were loaded on the Biomeck 2000 instrument.

After real-time PCR for HPV-16, none of the 24 negative samples gave a positive signal, while all 24 positive samples gave a nice amplification plot results indicating that no cross contamination had taken place during the automated procedure (data not shown).

Real-time PCR assays compared to GP+- PCR/sequencing system and INNO-LiPA HPV Genotyping

A total of 296 samples were tested in parallel using GP+- PCR and sequencing system and TaqMan assays. Of these, 250 resulted GP+- PCR negative and 46 GP+- PCR positive. Important, to avoid problems associated with sensitivity limit only samples that were TaqMan positive with >104 copy/reaction were considered. Furthermore, DNA was extracted by the manual method and the real-time PCR assays were performed in a double-tube configuration. Overall, out of 46 samples positive by GP+- PCR and sequencing system, 2 specimens (one HPV-18 and one HPV-31) were not confirmed negative by Real-time PCR assays (Table 7).

**Table 7**. Comparison of results obtained by GP+- PCR/sequencing system and INNO-LiPA with Real-time PCR assays

| Method utilized (no. of samples analyzed) | HPV types identified | | | |
|---|---|---|---|---|
| | HPV-16 | HPV-31 | HPV-18/-45 | HPV-33 group |
| GP$^+$- PCR and sequencing (46)[a] | 22 | 10 | 6 | 8 |
| INNO-LiPA (31) | 10 | 10 | 3 | 8 |
| No. of cases not confirmed by TaqMan assay | 0/32 (0) | 2/20[b] | 1/9[c] | 1/16[d] |

[a] For the comparison with the TaqMan assay was considered only the samples resulted with >$10^4$ copy/reaction; [b]one of 10 samples HPV-31 positive by GP$^+$- PCR and of 10 samples HPV-31 positive by INNO-LiPA were not confirmed by TaqMan assay; [c]one of the five samples HPV-18 positive by GP$^+$- PCR was not confirmed by TaqMan assay; [d]one of the four samples HPV-58 positive by INNO-LiPA was not confirmed by TaqMan assay.

Moreover, 15 of the 250 samples GP$^+$- PCR negative samples were found positive by the TaqMan assay with a viral load > $10^3$ copies/reaction. Of these, six samples (one for HPV-16, one for HPV-31 and four for HPV-33 group) resulted positive by the TaqMan assay with a viral load > $10^4$ copies/reaction (up to sensitivity limit of the GP$^+$- PCR) and nine samples (five for HPV-16, two for HPV-18, one for HPV-31 and three for HPV-33 group) with a viral load from $10^3$ to $10^4$ copies/reaction. All samples GP+-PCR negative that were found positive by the TaqMan assys with a viral load > $10^4$ copies/reaction were confirmed by PCR with MY09/MY11 primes sets, as showed in table 8.

**Table 8.** Overview of discordant cases confirmed by MY-PCR

| #Sample | GP$^+$-PCR | MY-PCR | Viral load by Real-time PCR assays | | | |
|---|---|---|---|---|---|---|
| | | | HPV-16 | HPV-31 | HPV-18/-45 | HPV-33 group |
| #1 | - | + (16) | 10.050 | 0 | 0 | 0 |
| #2 | - | + (31) | 10.120 | 0 | 0 | 0 |
| #3 | - | + (52) | 0 | 10.120 | 0 | 0 |
| #4 | - | + (52) | 0 | 0 | 0 | 46.000 |
| #5 | - | + (52) | 0 | 0 | 0 | 37.800 |
| #6 | - | + (58) | 0 | 0 | 0 | 12.650 |

Overall, of the 296 specimens tested in parallel using -GP$^+$- PCR and sequencing system and TaqMan assays (288 of 296 (97.3%), = 0.91, 95% CI = 0.86 to 0.96) generated concordant results for oncogenic HPV presence or absence by the two assays. We found an excellent degree of agreement for HPV-16 (one only discordant case) between the results revealed by real-time PCR and that determined by conventional PCR.

Finally, out of 31 samples positives by INNO-LiPA positive only two samples (one HPV-31 and one HPV-58) wer not confirmed by TaqMan assay (table 8). Overall, only a total of 4 (5%) of 77 resulted positive by $GP^+$- PCR or INNO-LiPA analysis were not confirmed by real-time PCR assays.

**Experimental Section #2**

MATERIAL AND METHODS

*Patients and clinical samples*

The study was performed on total of 597 patients. Of these 472 women were visited in the gynaecological outpatients clinic at San Gerardo Hospital, Monza and had pathologically proven cancerous or precancerous cervical lesions: 105 atypical squamous cells of undetermined significance (ASCUS), 200 low-grade (L-SIL), 152 high-grade (H-SIL) squamous intraepithelial lesions and 15 cervical cancer (CC). The age of these patients ranged between 19-76 years (median 37). 125 cervical samples, obtained from women aged between 20-65 (median 50), were found to have normal cytology. All specimens were collected between February 2005 and December 2006. Participating women gave informed consent and the study was approved by the local ethical committee.

*Cytological and histological examination of samples*

A Pap test was performed on all cohort participants during clinical investigation. Smears were classified into five ordered categories (negative, ASCUS, L-SIL, H-SIL, or CC). Colposcopy was carried out on patients with a pathological Pap smear and a punch biopsy was taken from suspected areas of the cervix. All histological assessments were performed by an experienced pathologist. If colposcopy findings were normal, no biopsy was taken and the case was classified as absence of cervical intraepithelial neoplasm (CIN). When there were discrepancies between the histological and cytological findings, the worst result was regarded as the final diagnosis.

*Sample processing and DNA extraction*

Sample processing was performed as descripted in matherial and methods of the experimental section #1. DNA extraction was performed by a commercial kit for DNA extraction based on binding of nucleic acid to silica membrane (Macherey-Nagel Nucleospin robot 96 blood kit). The 96-well plate format of the commercially available Macherey-Nagel Nucleospin kit allow integration into the workstation of a robotic liquid handler (Biomeck 2000, Beckman) as reported in material and methods of the experimental section #1.

*Quantification of HPV-DNA by Real-time quantitative PCR assays*

High-risk HPV DNA loads were quantified by the use of five independent real-time quantitative TaqMan PCR assays: HPV-16, 18, 31, 45 and 33 group. Details about these assays are described in the section #1. A CCR5 quantitative detection system was also used to quantify human genomic DNA in each sample and to normalize the viral load (Broccolo et al., 2005). The viral load is expressed as copy number for 104 cells.

*Statistical analysis*

The Cochran-Armitage Trend test was used to detect an increasing trend in the proportion of HPV positive samples from women with normal cytology to those with CC. $\chi$2 test was used to analyze the significance of the different prevalence of HPV genotypes in cervical samples from patients and controls.

Further statistical analyses were then conducted to evaluate: i) the difference in oncogenic HPV viral load between women with normal cytology and women with cervical prencancerous and cancerous lesions; ii) the association between HPV viral load and the severity of cervical lesions; iii) the association of HPV load with the risk of developing cancerous lesions; iv) the relationship between viral load and severity of disease in terms of sensitivity and specificity for high grade displasia.

The two-tailed Student t test was used to evaluate the significance of differences in oncogenic HPV load between women with normal cytology (reference group) and women with cervical prencancerous and cancerous lesions (test group). The total viral load, defined as the sum of the viral load for each type of virus where multiple viruses were found to be present, was calculated for each woman.

An index of cograduation ($\gamma$ Goodman and Kruskall's index) was used to ordinal variables. This test was also applied to measure the association grade (cograduation) between levels of DNA for each genotype and the severity of lesions. Goodman and Kruskall's index ($\gamma$) less than 0.3 represents fair to poor association, values of more than 0.3 represents good association. The level of statistical significance was set 0.05 and SPSS version 13 was used for all analyses.

To determine the association between the risk of cervical disease and HPV viral load, age adjusted odds ratios (AOR) were estimated by polytomous logistic regression. Type-specific viral load thresholds were defined by using the median values of viral load. These values were subsequently used to determine the AOR and 95% confidence intervals (CI). AOR and 95% CI were estimated for categories of cervical cytology, adjusting for the potential confounders of age.

A nonparametric receiver operating characteristic (ROC) curve was generated for each histological status using Stata7. Clinical sensitivity and specificity were computed at each cut-point as well the percentage of correctly classified subjects. The ordinal classification method, based on categorical viral load, was compared to dichotomous classification method via a test for the equality of the areas under the ROC curves. Both total viral load and specific HPV viral load were considered. For each observation, the histological and cytological findings was set as normal or positive and compared with the classification value of 0= "negative", 1="Low viral load" and 2="High viral load" considering as cut-off points the absence of HPV genomes, a HPV genomes per cell equivalent viral load lower or upper the median value, respectively.

HPV Viral load values obtained from the duplicate tests were averaged for calculations. Normalization of HPV type-specific viral load was calculated as:

$$VL = \frac{Cn_{HPV}}{(Cn_{CCR5}/2)} \times 10^{4} \text{ cells}$$

where VL is the number of HPV genomes per $10^4$ cells (corresponding to 2x $10^4$ CCR5 copies, $Cn_{HPV}$ is the number of HPV genomes and $Cn_{CCR5}/2$ is the number of cells.

**Results**

*Prevalence of the six oncogenic genotypes in pathological and normal cervical samples*

Real-time PCR quantification assays were used to investigate the frequency of type-specific HPV infection in an area of northen Italy. Positivity for one or more of the eight oncogenic HPV genotypes studied was found in 288 (61%) of 472 and in 32 (26%) of 125 respectively of the pathological and normal cervical samples analysed (Table 9). In pathological samples the overall prevalence of HPV-16, -31, -18, -45, and -33 group (including 33, 52, 58, 67) was 24%, 23%, 12%, 1%, 29%, respectively. As expected the prevalence of HPV types studied was markedly lower in normal samples with respect to each pathological category analyzed ($p < 0.05$) (Table 9). The presence of HPV genotypes studied was progressively higher in patients with ASCUS, L-SIL, H-SIL and CC as compared to women with normal cytology for all genotypes (P trend <0.05) except for types belonging to HPV-33 group (Table 7). Overall, 417 positive tests for oncogenic HPV-DNA were detected in patients with precancerous and cancerous lesions; of these 115 (27.6%) were positive for HPV-16, 55 (13.2%) for HPV-18, 108 (25.9%) for HPV-31, 3 (1.2%) for HPV-45 and 134 (32.6%) for HPV-33 group (table 9).

**TABLE 9**. Frequency of HPV16, -18, -45, -31, -33, and/or 52, -58, -67 in cervical samples from patients with diagnosis of ASCUS, LSIL, HSIL, cervical carcinoma and women with normal citology

| Diagnosis[a] | No. of patients | No. of cases of HPV-DNA positive for (%): | | | | | No. of patients positive for one or more genotypes (%) | P[d] |
|---|---|---|---|---|---|---|---|---|
| | | HPV-16 | HPV-18 | HPV-31 | HPV-45 | HPV-33 group | | |
| Normal | 125 | 9 (7) | 3 (2) | 17(14) | 0 (0) | 19 (15) | 32 (26) | 0.003 |
| ASCUS | 105 | 17 (16) | 12 (11) | 19 (18) | 0 (0) | 35 (33) | 59 (56) | 0.006 |
| LSIL | 200 | 34 (17) | 22 (11) | 47 (24) | 2 (1) | 57 (28) | 99 (50) | 0.006 |
| HSIL | 152 | 58 (38) | 21 (14) | 38 (25) | 2 (1) | 39 (26) | 118 (78) | 0.006 |

(continued)

| Diagnosis[a] | No. of patients | No. of cases of HPV-DNA positive for (%): | | | | | No. of patients positive for one or more genotypes (%) | P[d] |
|---|---|---|---|---|---|---|---|---|
| | | HPV-16 | HPV-18 | HPV-31 | HPV-45 | HPV-33 group | | |
| CC | 15 | 6 (40) | 0 (0) | 4 (27) | 1 (7) | 3 (20) | 12 (80) | 0.006 |
| P value of Cochran-Armitage Trend test[c] | | <0.0001 | 0.002 | 0.0060 | | 0.1299 | <0.0001 | |

[a]ASCUS, atypical squamous cells of undetermined significance cells; L- or H-SIL, low- or high-grade squamous intraepithelial lesions; CC, cervical cancer; [b]The presence of one or more HPV genotypes was significantly higher in patients with ASCUS, LSIL, HSIL and CC as compared to women with normal cytology; [c]If statistically significant, the Cochran-Armitage Trend test detects an increasing trend in proportion of HPV positive from Normal to CC; [d]$\chi 2$ test was used to analyze the significance of the different HPV genotypes prevalence in cervical samples from patients and controls.

*HPV infections with multiple types*

The real-time PCR assays were also able to determine the presence of multiple infections, by the high risk HPV genotypes studied, which were overall found to be present in 22% of patients with cervical pathology (39% in ASCUS, 43% in L-SIL, 35% in H-SIL and 7% in of CC) and in 25% of women with normal cytology. Moreover, the prevalence of multiple infections with the oncogenic HPV genotypes analysed was found to be significantly higher in patients with pathological findings with respect to normal women (102 (22%) of 472 vs. 8 (6%) of 125; P=0.006). Presence of each HPV type, alone and/or in combination with others, in patients with normal and pathological categories is shown in Figure 3.

Type-specific HPV viral load associated with the different cytological/histological diagnosis

The viral loads for oncogenic HPVs types studied are summarized in Table 10.

**Table 10**. Type-speecific HPV viral load (copy number/ $10^4$ cells)

| HPV type | n | 25% ILE | Mean | Median | 75% ILE | range |
|---|---|---|---|---|---|---|
| 16 | 124 | 4.4 $x10^2$ | 4.3 $x10^6$ | 2.1 $x10^4$ | 1.9 x $10^5$ | 1 - 3.5 $x10^8$ |
| 18 | 58 | 1.3 $x10^2$ | 5.8 $x10^5$ | 9.3 $x10^2$ | 1.05 $x10^5$ | 10 -1.9 $x10^7$ |
| 31 | 125 | 14 | 4.1 $x10^6$ | 80 | 2.4 $x10^3$ | 3 -1.9 $x10^7$ |
| 33 group | 153 | 50 | 2.3$x10^6$ | 2.9$x10^2$ | 4.2 $x10^3$ | 11 - 2.9 $x10^7$ |
| 45 | 5 | 1.1 $x10^2$ | 3.2 $x10^5$ | 7.1 $x10^2$ | 1 $x10^5$ | 10 -1.3 $x10^7$ |
| Total viral load[a] | 115 | 80 | 6.9 $x10^5$ | 2.2 $x10^3$ | 1.08 $x10^5$ | 1 - 3.5 $x10^8$ |

[a]Total viral load defined as the sum of the viral load for each type of virus analysed where multiple viruses were found to be present

No significant differences in the mean viral load values of the different HPV types considered were observed (data not shown). As shown in figure 4, there was a linear increase in the median values of total viral loads for the HPV genotypes studied with disease progression.

Overall, there was found to be a good association between HPVs total viral loads and severity of cytopathological/histological findings ($\gamma$=0.46). When viral load for each individual assay was analyzed there was found to be a significantly higher DNA load for HPV-16 and -31 genotypes in patients with CC when compared to women with normal cytology ($P < 0.05$); by contrast no statistically significant difference was found for HPV-18, -45 and -33 group (figure 2). A significant higher association between viral load and severity of disease was observed for HPV-31 and HPV-16 ($\gamma$=0.49 and 0.41 respectively); a less significant association was found in the case of HPV-18 and 33 group ($\gamma$=0.19 and 0.02 respectively). Finally, it was interesting to note that in 4 (12.5%) of the 32 HPV positive women with normal cytology, the viral DNA load was found to be higher than $10^4$ copies/$10^4$ cells.

The AOR between HPV viral load and histological status is reported in table 11.

**Table 11.** Age adjusted Odds Ratios (95% CI) for ASCUS, LSIL and HSIL in relation to the normalised HPV viral load of individual HPV types or group of closely related HPV types

| Viral load of HPV types[1] | Normal | ASCUS | | | LSIL | | | HSIL | | | CC | | |
|---|---|---|---|---|---|---|---|---|---|---|---|---|---|
| | | n | OR | 95%CI | n | OR | 95%CI | n | OR | 95% CI | n | OR | 95% CI |
| HPV-16 | | | | | | | | | | | | | |
| Negative | 116 | 88 | 1.0 | | 166 | 1.0 | | 94 | 1.0 | | 9 | 1.0 | |
| Low (1-3072) | 7 | 12 | 2.60 | 0.91-7.45 | 21 | 26 | 0.91-7.04 | 21 | 3.95 | 1.39-11.24 | 1 | 1.94 | 0.16-23.38 |
| High (>3072) | 2 | 5 | 4.07 | 0.43-38.1 | 13 | 7.9 | 0.71-87.75 | 37 | 30.0 | 3.76-2.3938 | 5 | 45.38 | 3.4-58641 |
| p-trend | | | <0.05 | | | <0.01 | | | <0.01 | | | <0.01 | |
| HPV-31 | | | | | | | | | | | | | |
| Negative | 108 | 87 | 1.0 | | 153 | 1.0 | | 113 | 1.0 | | 11 | 1.0 | |
| Low (1-57) | 10 | 14 | 1.97 | 0.73-5.3 | 20 | 1.87 | 0.75-4.69 | 18 | 2.07 | 0.79-5.44 | 1 | 1.56 | 0.19-13.07 |
| High (>57) | 7 | 4 | 0.58 | 0.2-1.78 | 27 | 1.56 | 0.62-3.93 | 21 | 1.57 | 0.62-4.01 | 3 | 3.20 | 0.86-11.68 |
| p-trend | | | >0.05 | | | >0.05 | | | >0.05 | | | >0.05 | |
| HPV-18 | | | | | | | | | | | | | |
| Negative | 122 | 93 | 1.0 | | 178 | 1.0 | | 131 | 1.0 | | 15 | 1.0 | |
| Low (1-110) | 2 | 6 | 1.97 | 0.45-8.65 | 15 | 231 | 0.57-9.41 | 8 | 27 | 0.79-9.15 | 0 | ND | |
| High (>110) | 1 | 6 | 4.67 | 0.59-37.0 | 7 | 2.93 | 0.35-24.72 | 13 | 8.0 | 1.32-4824 | 0 | ND | 0.43-46.59 |
| p-trend | | | >0.05 | | | >0.05 | | | <0.01 | | | ND | |
| HPV-33 group[2] | | | | | | | | | | | | | |
| Negative | 106 | 70 | 1.0 | | 136 | 1.0 | | 112 | 1.0 | | 12 | 1.0 | |
| Low (1-92) | 7 | 19 | 4.6 | 1.61 - 13.2 | 31 | 5.49 | 1.92-15.71 | 20 | 4.02 | 1.41-11.43 | 1 | 151 | 0.15-14.84 |
| High (>92) | 12 | 16 | 1.71 | 0.71- 4.08 | 28 | 1.54 | 0.68 - 3.48 | 19 | 1.26 | 0.53-3.0 | 2 | 1.27 | 0.23-7.11 |
| p-trend | | | <0.05 | | | <0.05 | | | >0.05 | | | <0.05 | |
| Total viral load | | | | | | | | | | | | | |
| Negative | 88 | 48 | 1.0 | | 88 | 1.0 | | 36 | 1.0 | | 4 | 1.0 | |
| Low (1-1.307) | 27 | 41 | 2.74 | 1.44 - 5.21 | 54 | 2.23 | 1.19-4.19 | 42 | 4.01 | 1.99-8.07 | 2 | 216 | 0.31-14.89 |
| High (>1.307) | 10 | 16 | 2.58 | 1.02 - 6.54 | 58 | 4.53 | 1.96-10.47 | 74 | 14.62 | 5.58-38.26 | 9 | 1574 | 3.79-65.37 |
| p-trend | | | <0.01 | | | 0.01 | | | 0.01 | | | <0.01 | |

AOR: Adjusted odds ratio (95% CI) estimated from polytomous logistic regression model; ND: not determined. [1]Based on median of viral load for HPV-16, -31, -18 and 33 group positive participants. [2]Include HPV-33, -52, -58 and -67.

The analysis was performed both for each HPV specific categorical level of viral load and for the total viral load irrespective of the HPV type. Overall, low and high levels of viral load were associated with risk of abnormal cytology. In particular, for low and high levels of total viral load, the AOR for ASCUS, L-SIL, H-SIL and CC were 2.7 (95%CI: 1.4-5.2) and 2.6 (95%CI: 1.0-6.5), 2.2 (95%CI: 1.2-4.2) and 4.5 (95%CI: 2.0-10.5), 4.0 (95%CI: 2.0-8.1) and 14.6 (95%CI: 5.6-38.3), 2.2 (95%CI: 0.3-14.9) and 15.7 (95%CI: 3.8-65.4), respectively (P-trend <0.01). In the case of HPV specific categorical level of viral load, high levels of HPV-16 viral load were strongly associated with risk of ASCUS, L-SIL, H-SIL and CC, ranging from 4.1 to 45.4 with a significant trend (P<0.05) across all categorical levels of viral load. Likewise, increased risk of abnormal cytology was also observed for HPV-18 high viral load, with a strong association between H-SIL and high viral load (AOR: 8.0; 95%CI: 1.3-48.2). A significant association was also observed between HPV-33 low viral load and ASCUS, L-SIL and H-SIL (AORs: 4.6, 5.5, 4.0; 95%CIs 1.6-13.2, 1.9-15.7, 1.4-11.4, respectively).

Finally, when considering total viral load, the areas under the nonparametric ROC curve .rise with the grading of histological status, ranging from 0.624 (95% CI: 0.560-0.688) for ASCUS to 0.778 (95% CI: 0.638-0.918) for CC (Table 12).

**Tab. 12.** Nonparametric receiver operating characteristic (ROC) curves for total viral load by grading of histological status

| Histological status | ROC Area | Std. Error | Asymptotic Normal [95% Conf. Interval] | |
|---|---|---|---|---|
| ASCUS | 0.624 | 0.033 | 0.560 | 0.688 |
| L-SIL | 0.652 | 0.027 | 0.599 | 0.705 |
| H-SIL | 0.775 | 0.026 | 0.724 | 0.827 |
| CC | 0.778 | 0.071 | 0.638 | 0.918 |

In the case of CC, the resulting sensitivity and specificity for the cut-off point 2 (High viral load) were 60% and 92%, respectively, with 88.6% of correctly classified subjects (data not shown). This percentage rose up to 91.4% when only categorical HPV-16 viral load was used as a classification cut-off point. Categorical viral load classification method significantly differed from the dichotomous one both for L-SIL and H-SIL (P <0.0001) (Figure 5). Although the areas under the ROC curves of categorical viral load classification method were higher also for ASCUS and CC when compared to the dichotomous method, the chi square tests yielded a significance probability higher than 0.05.

**Description of the Figures**

**Figure 1.** Standard curve and Amplification plot for HPV-16 (A and B) and -31 (C and D). Dilution series were made with plasmids containing 10° to $10^6$ HPV-16 and HPV-31 copies. The threshold cycle ($C_t$) number is plotted against number of HPV-16 copies.

**Figure 2.** Comparison of the reference curves for the HPV-18 and -45 (A) and for HPV-33, -52/-58/-67 (B) TaqMan assays. The number of the plasmid molecules containing HPV are indicated on the x axis, and the cycle of threshold (Ct) is indicated on the y axis; open triangles, for HPV-18 (A) and HPV-33 (B) TaqMan assays; open diamond, for HPV-45 (A) and HPV-52 (B); open circle, for HPV-58 (B); open squared, foe HPV-67. The equations for the HPV-18 and the HPV-45 constructs were y =38.83.- 3.32 log (x) and y =39.89 - 3.48 log (x), respectively. The equations for the HPV-33 and the HPV-52,-58,-67 constructs were y =38.93.- 3.34 log (x) and y =39.02 - 3.41 log (x), y =39.42 - 3.45 log (x), y =39.12 - 3.36 log (x), respectively.

**Figure 3.** Prevalence of oncogenic HPV alone and in combination with others genotypes.

**Figure 4.** Distribution of HPV viral load, according to the degree of cervical lesion or cytological finding (normal, ASCUS, L-SIL, H-SIL and CC). Each box indicates the interquartile range. The lines that extend from each box indicate the extremes of values (whiskers represent the extreme values), and the line across each box indicates the median. The viral: load was expressed in a log scale. A good association was showed between total oncogenic HPVs viral load and lesion degree ($\gamma$=0.46; figure A) as well as for HPV-16 ($\gamma$=0.41; B) and HPV-31 ($\gamma$=0.49; C); by contrast poor or none association was found for HPV18 or 45 ($\gamma$=0.19; D) and HPV-33 or 58 ($\gamma$=0.02; E).

**Figure 5.** Comparison between ROC curves for H-SIL via dichotomous and polytomous viral load.

## REFERENCES

1. Andersson S et al. 2005 Br J Cancer: 92:2195-200.
2. Badaracco G et al. 2002. J Med Virol.: 67: 574-582.
3. Beskow AH et al. 2002. Int J Cancer: 101(6):526-31
4. Bosch FX et al. 2002. J Clin Pathol.: 55:244-65.
5. Bosch FX et al. 1995. J Natl Cancer Inst.: 87: 796-802.
6. Bosch FX et al. 2002. Virus Res.: 89: 183-190.
7. Boshart M et al. 1984. EMBO J.: 3: 1151- 1157.
8. Broccolo F et al. 2005. J Invest Dermatol. 124:1234-40.
9. Cullen AP et al. 1991. J Virol.: 65: 606-612.
10. Cuschieri KS et al. 2004. J Med Virol 2004;73:65-70.
11. Cuzick J et al. Br J Cancer: 69: 167-71.
12. Dalstein V et al. 2003. Int J Cancer: 106: 396-403.
13. Daniel B et al. J Gen Virol.: 78(Part 5): 1095-1101.
14. De Francesco MA et al. 2005. J Med Virol.: 75(4):588-92.
15. Flores R et al. 2006. Int J Cancer: 118: 1187-93.
16. Gravitt PE et al. J Virol Methods: 2003, 112(1-2):23-33.
17. Gravitt PE et al. 2003. Cancer Epidemiol Biomarkers Prev.:12: 477-484.
18. Gravitt PE et al. 2003. J Virol Methods: 112(1-2):23-33.
19. Hart KW et al. 2001. J Clin Microbiol.: 39(9):3204-12.
20. Heid CA et al. 1996. Genome Res.: 6:986-994.
21. Hesselink AT, et al. 2005. J Clin Microbiol. Sep;43(9):4868-71.
22. Ho GY,et al. 1998. N Engl J Med.: 338: 423-428.
23. Hudelist G, et al. 2004. Gynecol Oncol.: 92: 873-880.
24. Jacobs MV, et al. 1995. J Clin Microbiol 33:901-5.
25. Jacobs MV, et al. 2000. Int J Cancer 87: 221-227.
26. Josefsson AM, et al. 1999. J Clin Microbiol.: 37: 490-496.
27. Josefsson AM, et al. 2000. Lancet 355: 2189-2193.
28. Kjaer SK, et al. 2002. BMJ. 14;325(7364):572.
29. Kraus I, et al. 2006. J Clin Microbiol. 44(4):1310-7.
30. Lorincz AT, et al. 2002. Lancet 360: 228-229.
31. Moberg M, et al. 2004. Int J Cancer: 112:854-9.
32. Moberg M et al. 2003. J Clin Microbiol. 41(7):3221-8.
33. Moberg M et al. 2004. Int J Cancer: 112: 854 -859.
34. Moberg M et al. 2005. Br J Cancer: 92: 891- 894.
35. Molden T et al. 2005. Cancer Epidemiol Biomarkers Prev 14 (2): 367-372.
36. Molden T et al. 2007. J Virol Methods;142(1-2):204-12.
37. Nagao S et al. 2002. J Clin Microbiol. 40(3):863-7.
38. Peitsaro P et al. 2002. J Clin Microbiol.: 40: 886-891.
39. Pirami L et al. 1997. J Clin Pathol.: 50: 600-604.
40. Rasu M et al. 2005. Scand J Infect Dis;37:476-481.
41. Roden R, Wu TC. 2006. Nature Reviews Cancer;6:753-763.
42. Schiffman MH et al. 1993. J Natl Cancer Inst.: 85: 958-964.
43. Schlecht NF et al. 2003. J Natl Cancer Inst.: 95: 1336-1343.
44. Sherman ME et al., 2002. J Natl Cancer Inst.: 94: 102-107.
45. Sherman ME et al. 2003. Cancer Epidemiol Biomarkers Prev.: 12: 1038-1044.
46. Sun CA et al. 2002. Int J Gynaecol Obstet.: 76: 41-47.
47. Swan DC et al. 1997. J Clin Microbiol: 35: 886-91.
48. Swan DC et al. 1999. J Clin Microbiol: 37: 1030-4.
49. van Duin M et al. 2002. Int J Cancer 98: 590- 595.
50. Walboomers JM et al. 1999. J Pathol: 189: 12-19.
51. Wang-Johanning F et al. 2002. Cancer: 94:2199-210.
52. Ylitalo N et al. 2000. Cancer Res 60: 6027-6032.
53. Woodman CBJ et al. 2007. Nature Reviews Cancer: 7:11-22.
54. Zerbini M et al. 2001. J Clin Pathol: 54:377-380.
55. Zur Hausen H. 2002.Nat Rev Cancer:2:342-50.

## Claims

**1.** A method for the identification and quantification of oncogenic HPV nucleic acids comprising:

a) first line screening by means of 5 independent SYBR Green I Realtime PCR assays to determine the total viral load and to identify the presence of one or more of 13 high risk HPV genotypes in the sample; wherein the primers used in step a) have the sequences SEQ IDs NO: 1-8;
b) second line assays to be applied to samples which have proved to be positive in the first line screening:

- 5 independent TaqMan Real-time PCR assays to determine the presence and the viral load of the most common oncogenic HPV types:

HPV types: 16, 18, 31, 45, 33 group (including 33, 52, 58, 67 genotypes)
wherein the primers used in the TaqMan Real-Time PCR assay have the sequences SEQ IDs 3, 4, 7, 8, 21-31,

- 6 independent SYBR Green I RT Real-time PCR assays to determine the presence in the sample of the oncogenic transcripts E6/E7 of HPV types 16, 18, 31, 33,45,58

wherein the primers used in the SYBR Green Real-time PCR assay in step b have the sequences SEQ IDs 9-20.

**2.** A method according to claim 1 wherein said sample can be any biological specimens such as cervical cytological samples, peripheral blood, urine, tissue biopsies and the like.

**3.** A kit for performing the methods of claims 1-or 2 including the primers of claim 1 and suitable buffers, probes and labels.

## Patentansprüche

**1.** Verfahren zur Identifizierung und Quantifizierung von onkogenen HPV-Nucleinsäuren umfassend:

a) erstes Screening mittels 5 unabhängigen SYBR-Green-I-Echtzeit-PCR-Tests, um die absolute Virenlast zu bestimmen und um die Gegenwart von einem oder meheren von 13 Hochrisiko-HPV-Genotypen in der Probe zu bestimmen; wobei die Primer, die in Schritt a) verwendet werden, die Sequenzen SEQ ID NOs:1 bis 8 haben;
b) zweite Assays, die auf Proben angewendet werden, die im ersten Screening als positiv befunden wurden:

- 5 unabhängige TaqMan-Echtzeit-PCR-Tests, um die Gegenwart und die virale Last der üblichsten onkogenen HPV-Typen zu bestimmen:

HPV-Typen: 16, 18, 31, 45, 33 Gruppe (einschließend 33, 52, 58, 67 Genotypen),
wobei die Primer, die in dem TaqMan-Echtzeit-PCR-Test verwendet werden, die Sequenzen SEQ ID NOs:3, 4, 7, 8, 21 bis 31 haben,

- 6 unabhängige SYBR-Green-I-RT-Echtzeit-PCR-Tests, um die Gegenwart von onkogenen Transkripten E6/E7 von HPV-Typen 16, 18, 31, 33, 45, 58 in der Probe zu bestimmen,
wobei die Primer, die in dem SYBR-Green-Echtzeit-PCR-Test in Schritt b verwendet werden, die Sequenzen SEQ ID NOs:9 bis 20 haben.

2. Verfahren nach Anspruch 1, wobei die Probe jede biologische Probe sein kann, so wie zervikale zytologische Proben, peripheres Blut, Urin, Gewebsbiopsien und dergleichen.

3. Kit zur Durchführung der Verfahren von Anspruch 1 oder 2, der die Primer nach Anspruch 1 und geeignete Puffer, Sonden und Markierungen beinhaltet.

## Revendications

1. Procédé d’identification et de quantification d’acides nucléiques de papillomavirus humain (PVh) oncogène, comprenant :

   a) un premier criblage réalisé au moyen d’une série de cinq tests indépendants de PCR en temps réel avec SYBR Green I, afin de déterminer la charge virale totale et de détecter la présence, dans l’échantillon, de l’un ou de plusieurs des treize génotypes de PVh à haut risque,
   étant entendu que les amorces utilisées dans cette étape (a) présentent les séquences données en tant que Séquences N° 1 à 8 ;
   b) et une deuxième série de tests effectués sur les échantillons qui se sont révélés positifs dans le premier criblage :

   - cinq tests indépendants de PCR en temps réel par TaqMan, afin de détecter la présence, et de déterminer la charge virale correspondante, des types de PVh oncogène les plus courants, soit les types 16, 18, 31 et 45 de PVH et le groupe du type 33, comprenant les génotypes 33, 52, 58 et 67,
     étant entendu que les amorces utilisées dans ces tests de PCR en temps réel par TaqMan présentent les séquences données en tant que Séquences N° 3, 4, 7, 8 et 21 à 31,
   - et six tests indépendants de PCR en temps réel avec SYBR Green I, afin de détecter la présence, dans l’échantillon, des transcrits oncogènes E6/E7 des types 16, 18, 31, 33, 45 et 58 de PVh,
     étant entendu que les amorces utilisées dans les tests de PCR en temps réel avec SYBR Green I de cette étape (b) présentent les séquences données en tant que Séquences N° 9 à 20.

2. Procédé conforme à la revendication 1, dans lequel ledit échantillon peut être n’importe quel échantillon biologique, comme des échantillons pour cytologie cervicale (frottis cervicaux), du sang périphérique, de l’urine, des biopsies de tissu, et autres similaires.

3. Trousse servant à mettre en oeuvre un procédé conforme à la revendication 1 ou 2, comprenant les amorces indiquées dans la revendication 1 et des tampons, sondes et marqueurs appropriés.

**Figure 1**

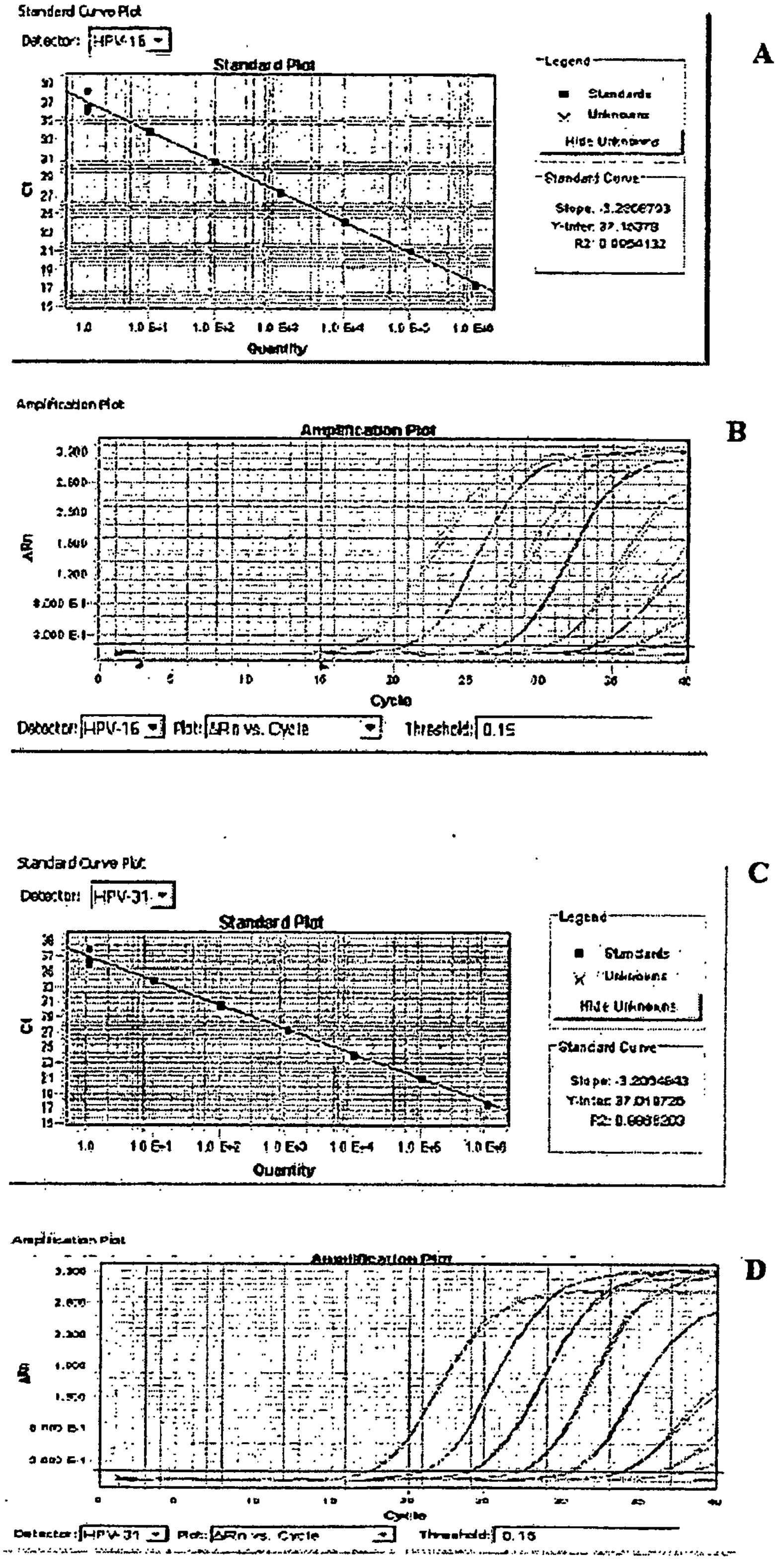
Standard Curve Plot
Detector: HPV-16
Standard Plot
Ct
Quantity
Legend
Standards
Unknowns
Hide Unknowns
Standard Curve
A
Amplification Plot
ΔRn
Cycle
Detector: HPV-16 Plot: ΔRn vs. Cycle Threshold: 0.15
B
Standard Curve Plot
Detector: HPV-31
Standard Plot
Ct
Quantity
Legend
Standards
Unknowns
Hide Unknowns
Standard Curve
C
Amplification Plot
Cycle
Detector: HPV-31 Plot: ΔRn vs. Cycle Threshold: 0.15
D

**Figure 2.**

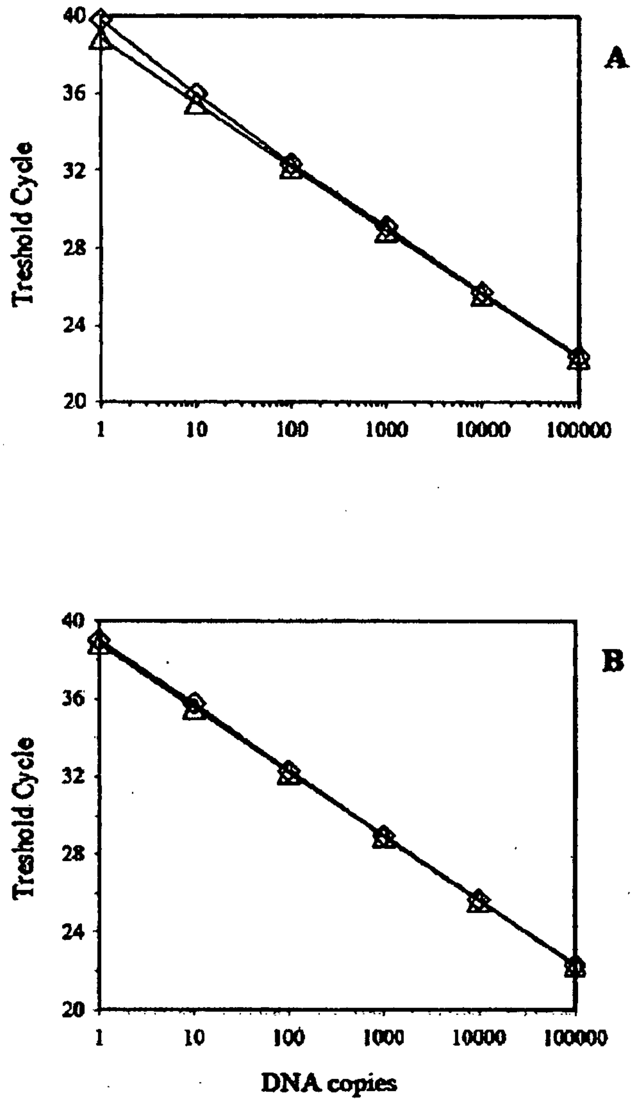
40
36
32
28
24
20
Treshold Cycle
1
10
100
1000
10000
100000
A
40
36
32
28
24
20
Treshold Cycle
1
10
100
1000
10000
100000
DNA copies
B

**Figure 3**

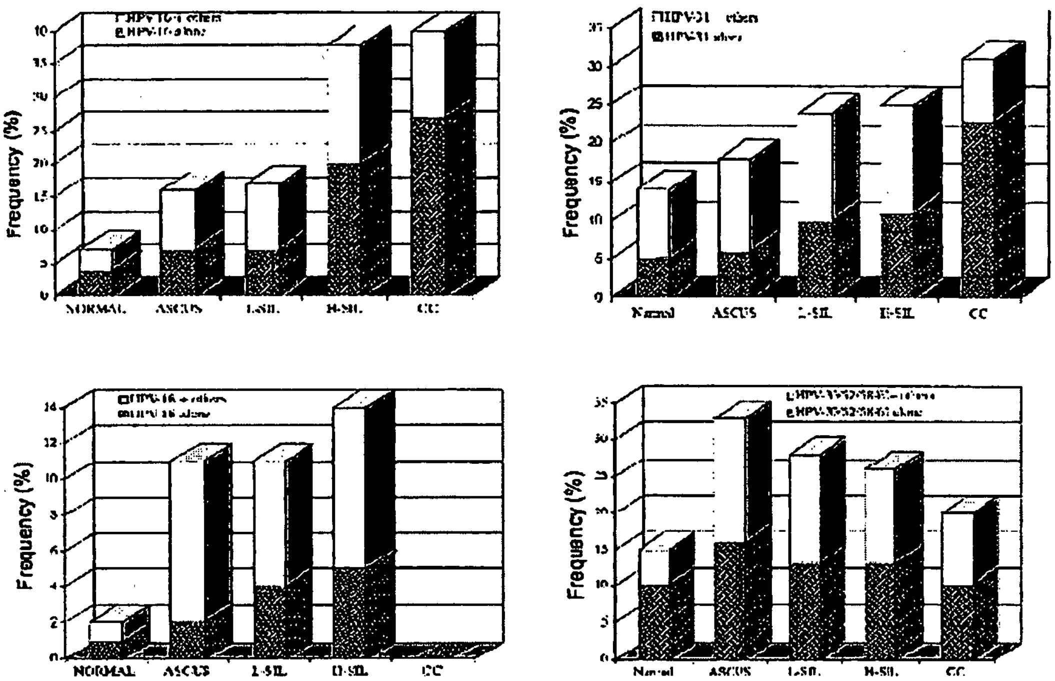
Frequency (%)
NORMAL
ASCUS
L-SIL
H-SIL
CC
Normal
ASCUS
L-SIL
H-SIL
CC

Figure 4.

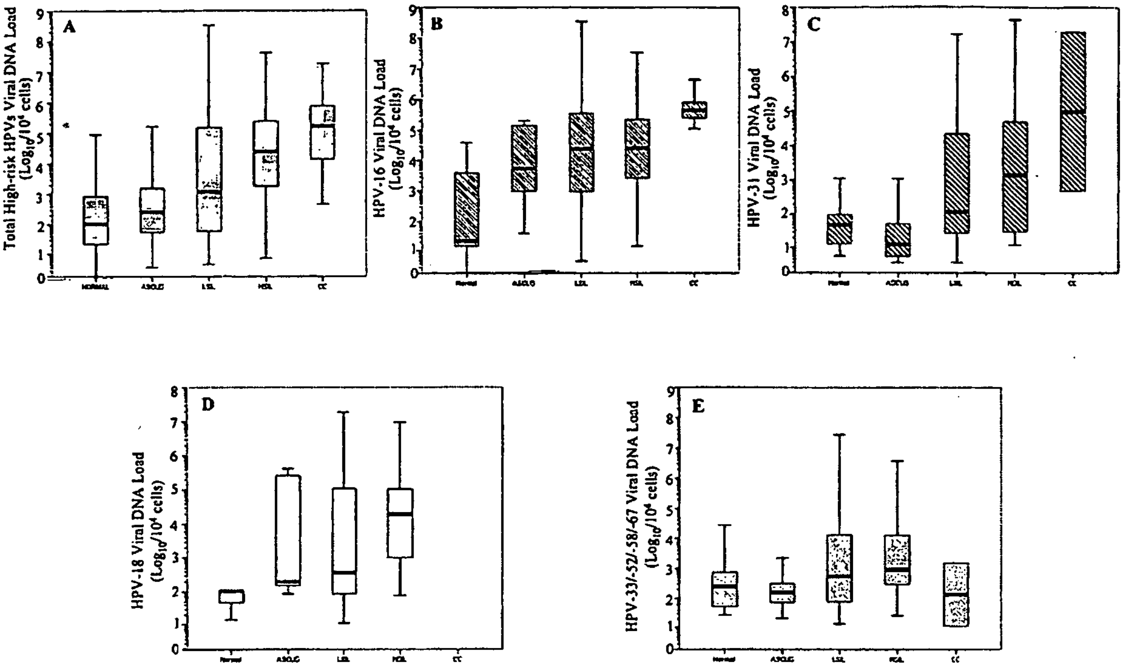
A
Total High-risk HPVs Viral DNA Load
($Log_{10}/10^4$ cells)
B
HPV-16 Viral DNA Load
($Log_{10}/10^4$ cells)
C
HPV-31 Viral DNA Load
($Log_{10}/10^4$ cells)
D
HPV-18 Viral DNA Load
($Log_{10}/10^4$ cells)
E
HPV-33/-52/-58/-67 Viral DNA Load
($Log_{10}/10^4$ cells)

**Figure 5**

| Classification method | Obs | ROC Area | Std. Error | Asymptotic Normal [95% Conf. Interval] | |
|---|---|---|---|---|---|
| Polytomous | 277 | 0.775 | 0.026 | 0.724 | 0.827 |
| Dichotomous | 277 | 0.516 | 0.030 | 0.456 | 0.574 |

Area Polytomous = Area Dichotomous
$\chi2$ (1) = 40.74 Prob > $\chi2$ = 0.0000

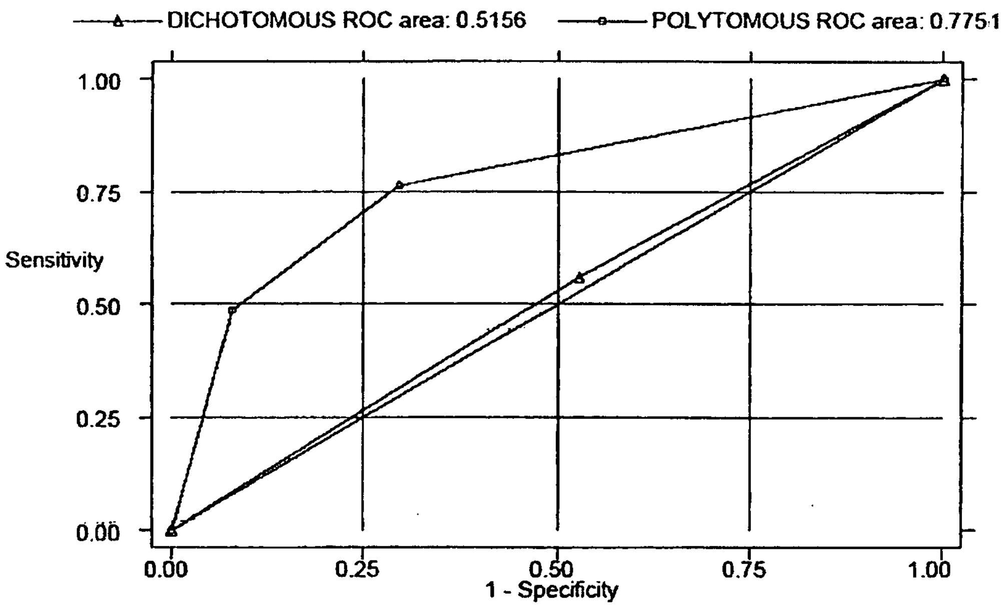

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004031416 A, Gyllesten
- EP 2008004277 W
- EP 07109454 A


### Non-patent literature cited in the description


- **SUN ZHEN et al.** *Genomics,* 2007, vol. 89 (1), 151-159
- **MACKAY et al.** *Nucleic Acid Research,* 2002, vol. 30 (6), 1292-1305
- **ANDERSSON S et al.** *Br J Cancer,* 2005, vol. 92, 2195-200
- **BADARACCO G et al.** *J Med Virol.,* 2002, vol. 67, 574-582
- **BESKOW AH et al.** *Int J Cancer,* 2002, vol. 101, 526-31
- **BOSCH FX et al.** *J Clin Pathol.,* 2002, vol. 55, 244-65
- **BOSCH FX et al.** *J Natl Cancer Inst.,* 1995, vol. 87, 796-802
- **BOSCH FX et al.** *Virus Res.,* 2002, vol. 89, 183-190
- **BOSHART M et al.** *EMBO J.,* 1984, vol. 3, 1151-1157
- **BROCCOLO F et al.** *J Invest Dermatol.,* 2005, vol. 124, 1234-40
- **CULLEN AP et al.** *J Virol,* 1991, vol. 65, 606-612
- **CUSCHIERI KS et al.** *J Med Virol,* 2004, vol. 73, 65-70
- **CUZICK J et al.** *Br J Cancer,* vol. 69, 167-71
- **DALSTEIN V et al.** *Int J Cancer,* 2003, vol. 106, 396-403
- **DANIEL B et al.** *J Gen Virol.,* vol. 78, 1095-1101
- **DE FRANCESCO MA et al.** *J Med Virol.,* 2005, vol. 75 (4), 588-92
- **FLORES R et al.** *Int J Cancer,* 2006, vol. 118, 1187-93
- **GRAVITT PE et al.** *J Virol Methods,* 2003, vol. 112 (1-2), 23-33
- **GRAVITT PE et al.** *Cancer Epidemiol Biomarkers Prev.,* 2003, vol. 12, 477-484
- **HART KW et al.** *J Clin Microbiol.,* 2001, vol. 39 (9), 3204-12
- **HEID CA et al.** *Genome Res.,* 1996, vol. 6, 986-994
- **HESSELINK AT et al.** *J Clin Microbiol.,* September 2005, vol. 43 (9), 4868-71
- **HO GY.** *N Engl J Med.,* 1998, vol. 338, 423-428
- **HUDELIST G et al.** *Gynecol Oncol.,* 2004, vol. 92, 873-880
- **JACOBS MV et al.** *J Clin Microbiol,* 1995, vol. 33, 901-5
- **JACOBS MV et al.** *Int J Cancer,* 2000, vol. 87, 221-227
- **JOSEFSSON AM et al.** *J Clin Microbiol.,* 1999, vol. 37, 490-496
- **JOSEFSSON AM et al.** *Lancet,* 2000, vol. 355, 2189-2193
- **KJAER SK et al.** *BMJ.,* 2002, vol. 325 (7364), 572
- **KRAUS I et al.** *J Clin Microbiol,* 2006, vol. 44 (4), 1310-7
- **LORINCZ AT et al.** *Lancet,* 2002, vol. 360, 228-229
- **MOBERG M et al.** *Int J Cancer,* 2004, vol. 112, 854-9
- **MOBERG M et al.** *J Clin Microbiol,* 2003, vol. 41, 3221-8
- **MOBERG M et al.** *Int J Cancer,* 2004, vol. 112, 854-859
- **MOBERG M et al.** *Br J Cancer,* 2005, vol. 92, 891-894
- **MOLDEN T et al.** *Cancer Epidemiol Biomarkers Prev,* 2005, vol. 14 (2), 367-372
- **MOLDEN T et al.** *J Virol Methods,* 2007, vol. 142 (1-2), 204-12
- **NAGAO S et al.** *J Clin Microbiol,* 2002, vol. 40 (3), 863-7
- **PEITSARO P et al.** *J Clin Microbiol.,* 2002, vol. 40, 886-891
- **PIRAMI L et al.** *J Clin Pathol.,* 1997, vol. 50, 600-604
- **RASU M et al.** *Scand J Infect Dis,* 2005, vol. 37, 476-481
- **RODEN R ; WU TC.** *Nature Reviews Cancer,* 2006, vol. 6, 753-763
- **SCHIFFMAN MH et al.** *J Natl Cancer Inst.,* 1993, vol. 85, 958-964

- **SCHLECHT NF et al.** *J Natl Cancer Inst.,* 2003, vol. 95, 1336-1343
- **SHERMAN ME et al.** *J Natl Cancer Inst.,* 2002, vol. 94, 102-107
- **SHERMAN ME et al.** *Cancer Epidemiol Biomarkers Prev.,* 2003, vol. 12, 1038-1044
- **SUN CA et al.** *Int J Gynaecol Obstet.,* 2002, vol. 76, 41-47
- **SWAN DC et al.** *J Clin Microbiol,* 1997, vol. 35, 886-91
- **SWAN DC et al.** *J Clin Microbiol,* 1999, vol. 37, 1030-4
- **VAN DUIN M et al.** *Int J Cancer,* 2002, vol. 98, 590-595
- **WALBOOMERS JM et al.** *J Pathol,* 1999, vol. 189, 12-19
- **WANG-JOHANNING F et al.** *Cancer,* 2002, vol. 94, 2199-210
- **YLITALO N et al.** *Cancer Res,* 2000, vol. 60, 6027-6032
- **WOODMAN CBJ et al.** *Nature Reviews Cancer,* 2007, vol. 7, 11-22
- **ZERBINI M et al.** *J Clin Pathol,* 2001, vol. 54, 377-380
- **ZUR HAUSEN H.** *Nat Rev Cancer,* 2002, vol. 2, 342-50

SEQUENCE LISTING

<110> UniversitS degli Studi di Milano-Bicocca

<120> Identification and quantification of oncogenic HPV nucleic acids

<130> PCT/EP2008/004277

<150> EP 07109454.4
<151> 2007-06-01

<160> 31

<170> PatentIn version 3.3

<210> 1
<211> 20
<212> DNA
<213> Artificial

<220>
<223> synthetic oligonucleotide

<400> 1
cgtccaaaag gaaactgagc 20

<210> 2
<211> 20
<212> DNA
<213> Artificial

<220>
<223> synthetic oligonucleotide

<400> 2
gcacagggac ataacaatgg 20

<210> 3
<211> 25
<212> DNA
<213> Artificial

<220>
<223> synthetic oligonucleotide

<400> 3
cgaaagtatt tgggtagtcc actta 25

<210> 4
<211> 30
<212> DNA
<213> Artificial

<220>
<223> synthetic oligonucleotide

<400> 4
cagctctact ttgtttttct atacatatgg 30

<210> 5
<211> 22
<212> DNA
<213> Artificial

<220>
<223> synthetic oligonucleotide

<400> 5
tttgaaagga catggtccag at 22

<210> 6
<211> 18
<212> DNA
<213> Artificial

<220>
<223> synthetic oligonucleotide

<400> 6
cgttccgaaa gggtttcc 18

<210> 7
<211> 19
<212> DNA
<213> Artificial

<220>
<223> synthetic oligonucleotide

<400> 7
ccaccacatc gaattccaa 19

<210> 8
<211> 16
<212> DNA
<213> Artificial

<220>
<223> synthetic oligonucleotide

<400> 8
cgccgcacac cttcac 16

<210> 9
<211> 29
<212> DNA
<213> Artificial

<220>
<223> synthetic oligonucleotide

<400> 9
cagagctgca aacaactata catgatata 29

<210> 10
<211> 24
<212> DNA
<213> Artificial

<220>
<223> synthetic oligonucleotide

<400> 10
gttaatacac ctcacgtcgc agta 24

<210> 11
<211> 19
<212> DNA
<213> Artificial

<220>
<223> synthetic oligonucleotide

<400> 11
ttacagaggt gcctgcggt 19

<210> 12
<211> 25
<212> DNA
<213> Artificial

<220>
<223> synthetic oligonucleotide

<400> 12
tctaagtttt tctgctggat tcaac 25

<210> 13
<211> 23
<212> DNA
<213> Artificial

<220>
<223> synthetic oligonucleotide

<400> 13
cattggaaat accctacgat gaa 23

<210> 14
<211> 24
<212> DNA
<213> Artificial

<220>
<223> synthetic oligonucleotide

<400> 14
ttgacacgtt atacaccttt gcag 24

<210> 15
<211> 22
<212> DNA

<213> Artificial

<220>
<223> synthetic oligonucleotide

<400> 15
ttgaactaca gtgcgtggaa tg 22

<210> 16
<211> 18
<212> DNA
<213> Artificial

<220>
<223> synthetic oligonucleotide

<400> 16
cagcgccctc agatcgtt 18

<210> 17
<211> 27
<212> DNA
<213> Artificial

<220>
<223> synthetic oligonucleotide

<400> 17
acaagacgta tctattgcct gtgtatat 27

<210> 18
<211> 17
<212> DNA
<213> Artificial

<220>
<223> synthetic oligonucleotide

<400> 18
ccgcaggcac ctctgtg 17

<210> 19
<211> 21
<212> DNA
<213> Artificial

<220>
<223> synthetic oligonucleotide

<400> 19
atcgaattga aatgcgttga a 21

<210> 20
<211> 17
<212> DNA
<213> Artificial

<220>

<223> synthetic oligonucleotide

<400> 20
gcacagcgcc ctcagat 17

<210> 21
<211> 20
<212> DNA
<213> Artificial

<220>
<223> synthetic oligonucleotide

<400> 21
agtgaatgtg tagacaataa 20

<210> 22
<211> 18
<212> DNA
<213> Artificial

<220>
<223> synthetic oligonucleotide

<400> 22
ttttgctgtg caaccgatt 18

<210> 23
<211> 22
<212> DNA
<213> Artificial

<220>
<223> synthetic oligonucleotide

<400> 23
agtgccagcg tactgtattg tg 22

<210> 24
<211> 16
<212> DNA
<213> Artificial

<220>
<223> synthetic oligonucleotide

<400> 24
cggttgcctt tggctt 16

<210> 25
<211> 17
<212> DNA
<213> Artificial

<220>
<223> synthetic oligonucleotide

<400> 25

cctgcgcctt gggcacc 17

<210> 26
<211> 20
<212> DNA
<213> Artificial

<220>
<223> synthetic oligonucleotide

<400> 26
cagtaccgag ggcagtgtaa 20

<210> 27
<211> 20
<212> DNA
<213> Artificial

<220>
<223> synthetic oligonucleotide

<400> 27
cgtctgcgaa gtctttcttg 20

<210> 28
<211> 17
<212> DNA
<213> Artificial

<220>
<223> synthetic oligonucleotide

<400> 28
acatgttgtg accaggc 17

<210> 29
<211> 22
<212> DNA
<213> Artificial

<220>
<223> synthetic oligonucleotide

<400> 29
ggacgtggtg caaattagat tt 22

<210> 30
<211> 22
<212> DNA
<213> Artificial

<220>
<223> synthetic oligonucleotide

<400> 30
gtgctgatat ttcctccatg gt 22

<210> 31

<211> 17
<212> DNA
<213> Artificial

<220>
<223> synthetic oligonucleotide

<400> 31
aggaagagga caaggaa 17